# EUROPEAN PATENT APPLICATION

(11) **EP 4 653 417 A1**
(43) Date of publication of application: **26.11.2025**
(21) Application number: 24744940.8
(22) Date of filing: 19.01.2024
(51) Int. Cl.: C07C 51/43, C07C 51/377, C07C 57/04, C07C 59/08

(54) **METHOD FOR VAPORIZING LACTIC ACID AND METHOD FOR PRODUCING ACRYLIC ACID**

(30) Priority: 20.01.2023 KR 20230008967
(71) Applicant: LG Chem, Ltd., Yeongdeungpo-gu Seoul 07336 (KR)
(72) Inventor: HONG, Daeho, Daejeon 34122 (KR); LEE, Jiyoung, Daejeon 34122 (KR); JEONG, Hoiin, Daejeon 34122 (KR); YEO, Gyeong Cheol, Seoul 07345 (KR); HWANG, Kyeong Hwan, Seoul 07345 (KR)
(74) Representative: Goddar, Heinz J.
(86) International application number: PCT/KR2024/001001
(87) International publication number: WO 2024/155161

(57) **Abstract**

The present disclosure relates to a method and an apparatus for vaporizing lactic acid, and a method for preparing acrylic acid from lactic acid. According to the method and apparatus of the present disclosure, the content of lactic acid oligomers can be reduced in a short period of time, and the content of single-molecule lactic acid can be increased.

## Description

### [TECHNICAL FIELD]

### CROSS-REFERENCE TO RELATED APPLICATION(S)

This application claims the benefits of Korean Patent Applications No. 10-2023-0008967 filed on January 20, 2023 and No. 10-2024-0008689 filed on January 19, 2024 with the Korean Intellectual Property Office, the disclosure of which is incorporated herein by reference in its entirety.

The present disclosure relates to a method for vaporizing lactic acid and a method for preparing acrylic acid from lactic acid.

### [BACKGROUND OF ART]

Lactic acid is also known as milk acid, or lactate, which is an organic acid with a relatively simple structure containing both a hydroxyl group and a carboxyl group in the molecule.

Lactic acid has traditionally been produced naturally during fermentation processes of lactose, glucose or the like, and has played a role in improving the flavor of fermented foods and is non-toxic to the human body. Thus, lactic acid has been widely used in the food field such as flavoring agents, preservatives, and pH regulators, in the cosmetic field such as moisturizing agents and skin whitening agents, or in the medical field such as intravenous injections, dialysis solutions, and calcium agents.

Poly(lactic acid) or polylactide is biodegradable, and thus recently attracts increasing attentions as an eco-friendly alternative polymer capable of replacing naturally non-decomposable plastics such as polyolefins, polystyrenes, and polyesters produced from petroleum. In addition, it is receiving great attention as a precursor of acrylic acid, which is considered very important industrially.

Lactic acid can be mainly produced by microbial fermentation or chemical synthesis. Recently, methods for producing lactic acid using biomass resources such as starch-based biomass including corn, sugar-based biomass including sugarcane, or cellulose-based biomass obtained from woody or herbaceous plants as raw materials are being studied.

The lactic acid obtained in this way is usually concentrated and stored at a high concentration during storage and distribution after production. However, due to the structural features peculiar to the lactic acid molecule containing both a hydroxyl group and a carboxyl group in the molecule, it often forms a dimer structure, or forms a dehydrated and condensed oligomer in which water molecules are removed.

A large amount of lactic acid is lost due to this dimerization or oligomerization. Oligomerized lactic acid has the problem of increasing the amount of by-products generated in chemical reactions using lactic acid and forming coking during the reaction process, thereby greatly reducing the efficiency of the reaction.

Therefore, when using the concentrated and stored lactic acid as lactic acid itself or adding it into other chemical reactions, it is necessary to reduce the content of lactic acid oligomers. It is known that the chemical equilibrium between lactic acid and lactic acid oligomers involves only the concentration of lactic acid, that is, the relative content ratio of lactic acid and water, and temperature. Since the equilibrium movement speed is very slow, there is growing interest in pretreatment methods for using concentrated lactic acid, i.e., methods of reducing the content of lactic acid oligomers in the feed and increasing the content of single-molecule lactic acid.

### [DETAILED DESCRIPTION OF THE INVENTION]

### [Technical Problem]

In the present disclosure, there is provided a vaporization method of lactic acid that can reduce the content of lactic acid oligomers in a short period of time and increase the content of single-molecule lactic acid.

Additionally, there is provided a method for preparing acrylic acid from the vaporized lactic acid.

### [Technical Solution]

In the present disclosure, there is provided a method for vaporizing lactic acid, including the steps of: mixing and spraying a first stream of a liquid phase containing a lactic acid aqueous solution and a second stream of a gas phase containing lactic acid vapor; vaporizing a lactic acid aqueous solution by heat exchange between the first stream and the second stream; and obtaining a third stream of a gas phase containing single-molecule lactic acid.

According to one aspect of the present disclosure, the lactic acid aqueous solution contained in the first stream may be concentrated to a high lactic acid concentration of about 20 to about 99 wt%, preferably about 20 wt% or more, about 25 wt% or more, about 30 wt% or more, or about 35 wt% or more, and about 99 wt% or less, about 95 wt% or less, about 90 wt% or less, or about 85 wt% or less.

Further, the lactic acid aqueous solution contained in the first stream may have a lactic acid multimer concentration, that is, a concentration of lactic acid oligomers of lactic acid dimer, trimer or higher of about 2 to about 80 wt%, preferably about 2 wt% or more, about 5 wt% or more, about 7 wt% or more, or about 8 wt% or more, and about 80 wt% or less, about 70 wt% or less, about 60 wt% or less, about 55 wt% or less, about 40 wt% or less, or about 20 wt% or less, which indicates that the content of multimers is relatively high.

According to another aspect of the present disclosure, the temperature of the first stream may be about 10 °C to about 200 °C, preferably about 10 °C or higher, about 15 °C or higher, or about 50 °C or higher, and about 200 °C or lower, about 180°C or lower, or about 150°C or lower.

According to one aspect of the present disclosure, the lactic acid vapor contained in the second stream may have a lactic acid concentration of about 20 to about 80 wt%, preferably about 20 wt% or more, about 25 wt% or more, about 30 wt% or more, or about 35 wt% or more, or about 80 wt% or less, about 70 wt% or less, about 60 wt% or less, about 55 wt% or less, about 50 wt% or less, or about 45 wt% or less.

In addition, the temperature of the second stream may be about 250 °C to about 400 °C, preferably about 250 °C or higher, about 270 °C or higher, or about 300 °C or higher, and about 400 °C or lower, about 380 °C or lower, or about 360°C or lower.

In addition, in the mixing and spraying step, the first stream flow rate: the second stream flow rate may be preferably about 1:2 to about 1:25.

According to one aspect of the present disclosure, at least some of the obtained third stream may be divided and recycled into the second stream.

Meanwhile, there is provided an apparatus for vaporizing lactic acid, including: a feed supply section configured to supply a first stream containing a lactic acid aqueous solution; a lactic acid vapor supply section configured to supply a second stream containing lactic acid vapor; a spray section configured to mix and spray a lactic acid aqueous solution supplied from the feed supply section and lactic acid vapor supplied from the lactic acid vapor supply section; a vaporization reaction section configured to vaporize the lactic acid aqueous solution after mixing and spraying; and an obtaining section configured to obtain a third stream containing vaporized lactic acid molecules.

At this time, the spray section may include a first nozzle through which the lactic acid aqueous solution is sprayed and a second nozzle through which the lactic acid vapor is sprayed, and the lactic acid aqueous solution droplets and the lactic acid vapor are mixed and sprayed simultaneously.

Meanwhile, there is provided a method for preparing acrylic acid, including the steps of: obtaining lactic acid molecules by vaporizing lactic acid according to any one of the above methods; subjecting the lactic acid molecules to a dehydration reaction to prepare acrylic acid; and obtaining the acrylic acid.

In the present disclosure, the terms "the first", "the second", and the like are used to describe a variety of components, and these terms are merely employed to distinguish a certain component from other components.

The terminology used herein is for the purpose of describing particular aspects only and is not intended to limit the disclosure.

The singular forms are intended to include the plural forms as well, unless the context clearly indicates otherwise.

It will be further understood that the terms "include", "have", or "possess" when used in this specification, specify the presence of stated features, numbers, steps, components, or combinations thereof, but do not preclude the presence or addition of one or more other features, numbers, steps, components, or combinations thereof.

Also, as used herein, when a layer or an element is mentioned to be formed "on" layers or elements, the layer or element may be directly formed on the layers or elements, or other layers or elements may be additionally formed between the layers, on a subject, or on a substrate.

As the present disclosure can be variously modified and have various forms, specific aspects thereof are shown by way of examples and will be described in detail. However, it is not intended to limit the present disclosure to the particular form disclosed and it should be understood that the present disclosure includes all modifications, equivalents, and replacements within the idea and technical scope of the present disclosure.

In the present disclosure, lactic acid is a compound represented by the following chemical formula, and is used to encompass all lactic acid isomers, naturally occurring lactic acid dimers, and lactic acid oligomers, unless otherwise specified herein.

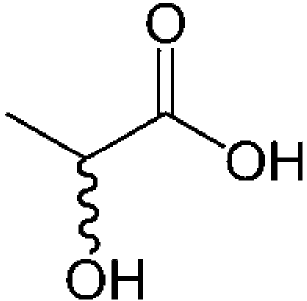

Hereinafter, the present disclosure will be described in detail.

According to one aspect of the present disclosure, there is provided a method for vaporizing lactic acid, including the steps of: mixing and spraying a first stream of a liquid phase containing a lactic acid aqueous solution and a second stream of a gas phase containing lactic acid vapor; vaporizing a lactic acid aqueous solution by heat exchange between the first stream and the second stream; and obtaining a third stream of a gas phase containing single-molecule lactic acid.

The present inventors have discovered that when a highly concentrated lactic acid aqueous solution is mixed and sprayed with high-temperature lactic acid vapor and vaporized through direct heat exchange, lactic acid can be efficiently vaporized in a very short period of time without increasing the oligomer concentration of lactic acid, and completed the present disclosure.

Lactic acid is widely used in the production of acrylic acid. When producing acrylic acid by dehydrating lactic acid, it is necessary to vaporize lactic acid into lactic acid molecules because it proceeds by a gas phase reaction.

However, as mentioned above, lactic acid is usually concentrated and stored at a high concentration during storage or distribution after production. Due to the structural feature peculiar to lactic acid molecules containing both hydroxyl groups and carboxyl groups in the molecule, it often forms a dimer structure, or forms a dehydrated and condensed oligomer in which water molecules are removed.

These lactic acid oligomer molecules are carbonized during the vaporization or reaction step to form coke, which may reduce the active area of the reaction catalyst and may also be included in the final product as a by-product. Above all, due to the presence of lactic acid oligomers, the content of lactic acid in the form of single molecules that can participate in the reaction is greatly reduced. Therefore, it is necessary to convert lactic acid oligomers into single molecules in the concentrated lactic acid aqueous solution, thereby lowering the oligomer content and increasing the content of single-molecule lactic acid.

However, when the concentration is diluted by simply adding water to the concentrated lactic acid aqueous solution containing a high content of lactic acid oligomers, the equilibrium movement speed is very slow, and thus, it takes a very long time to reduce the content of the lactic acid oligomers.

Generally, in order to shorten this time, a lactic acid aqueous solution with a high concentration of about 80 wt% or more is heated and vaporized. However, the vaporization efficiency of lactic acid is lower than that of water, so that the water is vaporized first, and then the proportion of lactic acid in the gas phase falls below about 20 wt%. In this case, as the water evaporates first, the concentration of the remaining lactic acid aqueous solution further increases. Accordingly, the concentration of oligomers in the remaining lactic acid aqueous solution also increases, which requires additional treatment.

The method for vaporizing lactic acid according to one aspect of the present disclosure includes the steps of: mixing and spraying a first stream of a liquid phase containing a lactic acid aqueous solution and a second stream of a gas phase containing lactic acid vapor; vaporizing a lactic acid aqueous solution by heat exchange between the first stream and the second stream; and obtaining a third stream of a gas phase containing single-molecule lactic acid.

That is, according to one aspect of the present disclosure, rather than directly heating and vaporizing a high-concentration lactic acid aqueous solution, the high-concentration lactic acid aqueous solution is sprayed in the form of an aerosol (or droplet), and high-temperature lactic acid vapor is mixed and sprayed therewith. Accordingly, lactic acid is vaporized through rapid heat exchange between high-concentration lactic acid droplets sprayed in the form of an aerosol and lactic acid vapor, making it possible to obtain vaporized single-molecule lactic acid.

In addition, a concentration of lactic acid in the third stream may be about 60 wt% or less, about 55 wt% or less, or about 50 wt% or less. The lower limit may not be significant depending on process conditions, and is about 0.1 wt% or more, or about 5 wt% or more.

According to one aspect of the present disclosure, the lactic acid aqueous solution contained in the first stream may be concentrated to a high lactic acid concentration of about 20 to about 99 wt%, preferably about 20 wt% or more, about 25 wt% or more, about 30 wt% or more, or about 35 wt% or more, and about 99 wt% or less, about 95 wt% or less, about 90 wt% or less, or about 85 wt% or less.

Further, the lactic acid aqueous solution contained in the first stream may have a lactic acid multimer concentration, that is, a concentration of lactic acid oligomers of lactic acid dimer, trimer or higher of about 2 to about 80 wt%, preferably about 2 wt% or more, about 5 wt% or more, about 7 wt% or more, or about 8 wt% or more, and about 80 wt% or less, about 70 wt% or less, about 60 wt% or less, about 55 wt% or less, about 40 wt% or less, or about 20 wt% or less, which indicates that the content of multimers is relatively high.

Herein, the lactic acid aqueous solution contained in the first stream, that is, the lactic acid feed in the process, is in a state in which lactic acid is dissolved in water, and as described above, naturally includes all single-molecule lactic acid, lactic acid dimers, and lactic acid oligomers depending on the temperature and concentration conditions. The lactic acid concentration described herein also refers to the concentration of lactic acid-based compounds including not only single-molecule lactic acid, but also single-molecule lactic acid, lactic acid dimers, and lactic acid oligomers.

More specifically, for example, when calculated by computer modeling based on the known oligomerization equilibrium constant of lactic acid, the oligomer content for each concentration of the total lactic acid-based compound is shown in Table 1 below.

However, this may have a slight calculation error depending on the actual calculation model, and the actual measured value may also have a slight measurement error depending on measurement conditions or measurement method (titration method or HPLC).

**[Table 1]**

| Concentration of total lactic acid-based compound (wt%) | Concentration of single molecule (wt%) | Concentration of dimer (wt%) | Concentration of trimer or higher (wt%) |
|---|---|---|---|
| 5 | 5.0 | 0.019 | 0.001 |
| 10 | 9.9 | 0.079 | 0.011 |
| 15 | 14.8 | 0.187 | 0.013 |
| 20 | 19.6 | 0.35 | 0.05 |
| 25 | 24.3 | 0.575 | 0.125 |
| 30 | 29.0 | 0.874 | 0.126 |
| 35 | 33.6 | 1.26 | 0.14 |
| 40 | 38.0 | 1.75 | 0.25 |
| 45 | 42.3 | 2.35 | 0.35 |
| 50 | 46.3 | 3.11 | 0.59 |
| 55 | 50.2 | 4.03 | 0.77 |
| 60 | 53.8 | 5.18 | 1.02 |
| 65 | 56.9 | 6.58 | 1.52 |
| 70 | 59.6 | 8.31 | 2.09 |
| 75 | 61.5 | 10.4 | 3.1 |
| 80 | 62.5 | 13.0 | 4.5 |
| 85 | 62.2 | 16.2 | 6.6 |
| 90 | 60.1 | 19.8 | 10.1 |
| 95 | 55.4 | 23.6 | 16 |
| 100 | 47.6 | 26.6 | 25.8 |

Additionally, the lactic acid aqueous solution supplied from the feed may be subjected to a heating process.

According to another aspect of the present disclosure, the temperature of the first stream may be about 10 °C to about 200 °C, preferably about 10 °C or higher, about 15 °C or higher, or about 50 °C or higher, and about 200 °C or lower, about 180°C or lower, or about 150°C or lower.

When the temperature of the first stream is too low outside the above temperature range, there may be a problem in which lactic acid is not sufficiently vaporized due to the low temperature of the mixture. When the temperature is too high, there may be a problem in that the pressure becomes excessively high to maintain the temperature.

The lactic acid aqueous solution supplied from the feed is heated and then sprayed in the form of droplets into the vaporization reactor through a transfer line and nozzle. The temperature of the lactic acid aqueous solution is kept high, and as the pressure is higher, it is more advantageous to obtain small-sized droplets.

At this time, separately from the first stream, a second stream containing lactic acid vapor is also mixed and sprayed with the first stream in the form of droplets into the vaporization reactor through a transfer line and nozzle.

At the time of mixing and spraying, the transfer lines of the first stream and the second stream may be first combined before spraying, and then mixed and sprayed through the same nozzle. Rather, it is more preferable that the first stream and the second stream are mixed and sprayed into the vaporization reactor through separate nozzles.

The second stream instantly lowers the concentration of lactic acid in the first stream containing highly concentrated lactic acid and simultaneously transfers heat energy to the first stream to promote vaporization of lactic acid molecules, thereby lowering the ratio of oligomers.

When using water vapor as a medium to transfer heat to lactic acid, a large amount of water vapor must be used to heat the lactic acid to the desired temperature, so there is a problem in that the lactic acid concentration in the vaporized product, that is, the vaporized lactic acid vapor, is lowered.

In addition, the temperature of the second stream may be about 250 °C to about 400 °C, preferably about 250 °C or higher, about 270 °C or higher, or about 300 °C or higher, and about 400 °C or lower, about 380 °C or lower, or about 360°C or lower.

When the temperature of the second stream is too low outside the above range, the lactic acid may not be sufficiently vaporized due to the low temperature of the mixture, and the lactic acid vapor may condense into a liquid. When the temperature of the second stream is too high outside the above range, the pressure may become excessively high to maintain the temperature, and a problem in which lactic acid in the lactic acid vapor is decomposed by heat may occur.

When the spray amount and spray rate of the first stream are too low, the flow rate of supplied lactic acid may become too low, causing problems of overreaction in the subsequent dehydration reaction, and when they are too high, a lot of heat is required for vaporization, thereby resulting in incomplete vaporization.

When the spray amount and spray rate of the second stream are too low, incomplete vaporization may occur because the heat supplied for vaporization is reduced, and when they are too high, the concentration of lactic acid may become diluted, which may cause problems in the subsequent product separation process.

At this time, in the mixing and spraying step, the first stream flow rate: the second stream flow rate may be preferably about 1:2 to about 1:25.

When the flow rate of the first stream is too low outside the above range, the flow rate of the supplied lactic acid becomes low, which may lead to lower efficiency and increased load during the product separation process. When the flow rate of the second stream is too low, the heat supplied for vaporization may be reduced and incomplete vaporization may occur.

According to another aspect of the present disclosure, before the first stream and the second stream are mixed and sprayed in the form of droplets into the vaporization reactor through the transfer line and nozzle, the inside of the vaporization reactor is preferably saturated with lactic acid vapor in advance.

When the first stream is sprayed into the reactor under high temperature conditions in this way, the phenomenon of water first evaporating and lactic acid concentrating in the droplets of the first stream instantaneously sprayed under high temperature conditions can be prevented.

At this time, it may be desirable to conduct the reaction in the vaporization reactor while maintaining the temperature of about 150 °C to about 350 °C.

Meanwhile, there is provided an apparatus for vaporizing lactic acid, including: a feed supply section configured to supply a first stream containing a lactic acid aqueous solution; a lactic acid vapor supply section configured to supply a second stream containing lactic acid vapor; a spray section configured to mix and spray a lactic acid aqueous solution supplied from the feed supply section and lactic acid vapor supplied from the lactic acid vapor supply section; a vaporization reaction section configured to vaporize the lactic acid aqueous solution after mixing and spraying; and an obtaining section configured to obtain a third stream containing vaporized lactic acid molecules.

At this time, the spray section may include a first nozzle through which the lactic acid aqueous solution is sprayed and a second nozzle through which the lactic acid vapor is sprayed, and the lactic acid aqueous solution droplets and the lactic acid vapor are mixed and sprayed simultaneously.

Meanwhile, there is provided a method for preparing acrylic acid, including the steps of: obtaining lactic acid molecules by vaporizing lactic acid according to the above method; subjecting the lactic acid molecules to a dehydration reaction to prepare acrylic acid; and obtaining the acrylic acid.

The reaction for producing acrylic acid by intramolecular dehydration of lactic acid can be represented by the following chemical formula, and is known to proceed in one step in the presence of a catalyst.

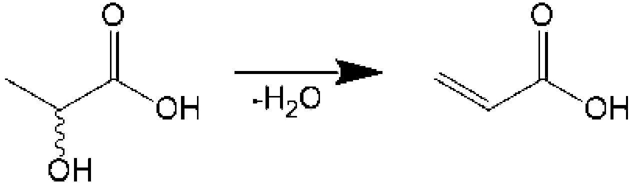

However, in the case of the method for preparing acrylic acid according to one aspect of the present disclosure, the method for preparing acrylic acid from vaporized lactic acid can employ catalysts, reactors, or reaction conditions generally known in the technical field to which the present disclosure pertains, except for the content related to the vaporization of lactic acid described above.

For example, the catalyst used in the dehydration reaction of lactic acid may be CaSO₄/ Na₂SO₄; Na₄P₂O₇/CaSO₄; Na₄P₂O_{y}/Ca₃(PO₄)₂; NaH₂PO₄-NaHCO₃/SiO₂; AlPO₄-NH₃; Ca₃(PO₄)₂/CaSO_{4,} and the like.

The dehydration reaction using a solid catalyst can be carried out as a continuous reaction using a fixed reactor, as a batch reaction, or the like. When using a fixed reactor, products can be produced continuously by charging the reactor with a solid catalyst and continuously supplying the reactants to the reactor for reaction.

The temperature of the dehydration reaction may be about 300 °C to about 500 °C, or about 300 °C to about 400 °C. The reaction pressure may be about 1 atmosphere to about 5 atmospheres, or about 1 atmosphere to about 2 atmospheres.

The supply velocity of reactants may vary depending on the type of reactor, other reaction conditions, etc. Specifically, for example, the gas phase lactic acid supply velocity (Weight Hourly Space Velocity, WHSV) may be about 0.05 to about 1.0/hr, or about 0.10 to about 0.50/hr.

When the above conditions are not met, a decomposition reaction due to oligomerization or hydrogenation may proceed, which may cause problems such as lowered reaction efficiency or lowered conversion rate.

### [ADVANTAGEOUS EFFECTS]

According to the method and device for vaporizing lactic acid according to one aspect of the present disclosure, it is possible to reduce the content of lactic acid oligomers in a short period of time and increase the content of single-molecule lactic acid in a high concentration lactic acid aqueous solution.

### [DETAILED DESCRIPTION OF THE EMBODIMENTS]

Hereinafter, the action and effect of the disclosure will be described in more detail with reference to specific examples of the disclosure. However, these examples are presented for illustrative purposes only and the scope of the disclosure is not limited thereby in any way.

### <Examples>

The lactic acid feed solution to be used as a first stream and the heat transfer medium to be used as a second stream were prepared under the conditions summarized in Table 2 below.

**[Table 2]**

| | Lactic acid feed solution | | Vaporization heat transfer medium | | | | note |
|---|---|---|---|---|---|---|---|
| | Concentration | Temperature | Type | Temp. | Press. | *amount of use | note |
| | wt% | °C | | °C | bar | wt% | |
| Example 1 | 30.0 | 130 | Lactic acid 30wt% vapor | 350 | 2.5 | 730 | Lactic acid vapor |
| Example 2 | 40.0 | 130 | Lactic acid 40wt% vapor | 350 | 2.5 | 730 | Lactic acid vapor |
| Example 3 | 40.0 | 130 | Lactic acid 40wt% vapor | 300 | 2.5 | 900 | Lactic acid vapor |
| Example 4 | 50.0 | 130 | Lactic acid 50wt% vapor | 350 | 2.5 | 670 | Lactic acid vapor |
| Example 5 | 60.0 | 130 | Lactic acid 60wt% vapor | 350 | 2.5 | 1150 | Lactic acid vapor |
| Example 6 | 80.0 | 130 | Lactic acid 80wt% vapor | 350 | 2.5 | 2340 | Lactic acid vapor |
| Comparative Example 1 | 40.0 | 174 | - | - | - | - | Heating |
| Comparative Example 2 | 40.0 | 201 | - | - | - | - | Heating |
| Comparative Example 3 | 80 | 130 | Steam | 400 | 2.5 | 250 | Steam |
| Comparative Example 4 | 80 | 130 | Steam | 400 | 2.5 | 380 | Steam |

| | | | | | | | |
|---|---|---|---|---|---|---|---|
| *Compared to 100 wt% of the flow rate of lactic acid aqueous solution supplied as first stream | | | | | | | |

Further, an apparatus for vaporizing lactic acid including: a feed supply section configured to supply a first stream containing a lactic acid aqueous solution; a lactic acid vapor supply section configured to supply a second stream containing lactic acid vapor; a spray section configured to mix and spray a lactic acid aqueous solution supplied from the feed supply section and lactic acid vapor supplied from the lactic acid vapor supply section through each nozzle; a vaporization reaction section configured to vaporize the lactic acid aqueous solution after mixing and spraying; and an obtaining section configured to obtain a third stream containing vaporized lactic acid molecules was prepared.

A thermometer and a temperature control device were provided in the feed supply section and the steam supply section, whereby the temperature of the supplied feed (first stream) and lactic acid vapor (second stream) can be adjusted. The obtaining section was equipped with a thermometer to measure the temperature of the third stream.

First, lactic acid vapor was introduced into the vaporization reactor so that the inside of the vaporization reactor was saturated with the lactic acid vapor.

Thereafter, the lactic acid aqueous solution was supplied to the first stream through the feed supply section, the lactic acid vapor was supplied through the lactic acid vapor supply section, and continuously mixed and sprayed into the inside of the vaporization reaction section through each nozzle. Thereby, the third stream containing vaporized lactic acid molecules was obtained through the obtaining section.

Some of the third stream was divided and supplied back to the second stream, and the unevaporated feed was supplied back to the first stream.

The obtained third stream was analyzed; and the concentration of the total lactic acid-based compound in the third stream and the proportion of oligomers among them were measured and calculated, which are summarized in the table below.

In Comparative Examples 1 and 2, a vaporization method of heating the vaporization reaction section was used without using a separate heat transfer medium.

**[Table 3]**

| | Produced lactic acid vapor | | | | note |
|---|---|---|---|---|---|
| | Concentration | Temperature | Pressure | Proportion of oligomers | |
| | wt% | °C | bar | wt% | |
| Example 1 | 29.5 | 240 | 2.5 | 4.8 | Lactic acid vapor |
| Example 2 | 38.6 | 245 | 2.5 | 6.3 | Lactic acid vapor |
| Example 3 | 39.2 | 218 | 2.5 | 6.7 | Lactic acid vapor |
| Example 4 | 49.1 | 236 | 2.5 | 9.2 | Lactic acid vapor |
| Example 5 | 57.8 | 274 | 2.5 | 13.3 | Lactic acid vapor |
| Example 6 | 75.3 | 317 | 2.5 | 29.4 | Lactic acid vapor |
| Comparative Example 1 | 22.8 | 174 | 1.0 | 11.1 | General heating |
| Comparative Example 2 | 35.3 | 201 | 1.0 | 31.5 | General heating |
| Comparative Example 3 | 24.1 | 257 | 2.5 | 15.4 | Steam |
| Comparative Example 4 | 16.1 | 243 | 2.5 | 12.9 | Steam |

Referring to the table above, it can be clearly confirmed that one aspect of the present disclosure can provide lactic acid vapor with a high concentration by continuously vaporizing lactic acid, while relatively reducing the content of lactic acid oligomers in the resulting product.

In the case of general heating, it was found that the temperature of the generated lactic acid vapor was low, the concentration of the lactic acid vapor was low, and the proportion of oligomers was relatively high despite the low temperature. In addition, when steam was used, the temperature of the generated lactic acid vapor increased, but there was a problem in that the proportion of oligomers was relatively high despite the low concentration of the lactic acid vapor.

## Claims

1. A method for vaporizing a lactic acid, comprising:
mixing and spraying a first stream in a liquid phase and containing a lactic acid aqueous solution and a second stream in a gas phase and containing a lactic acid vapor;
vaporizing the lactic acid aqueous solution by heat exchange between the first stream and the second stream; and
obtaining a third stream in a gas phase and containing a single-molecule lactic acid.

2. The method of claim 1,
wherein the lactic acid aqueous solution has a lactic acid concentration ranging from 20 wt% to 99 wt%.

3. The method of claim 1,
wherein the lactic acid aqueous solution has a lactic acid multimer concentration ranging from 2 wt% to 80 wt%.

4. The method of claim 1,
wherein a temperature of the first stream ranges from 10 °C to 200 °C.

5. The method of claim 1,
wherein the lactic acid vapor has a lactic acid concentration ranging from 20 wt% to 80 wt%

6. The method of claim 1,
wherein a temperature of the second stream ranges from 250°C to 400 °C.

7. The method of claim 1,
wherein in the mixing and spraying, a ratio of a first stream flow rate to a second stream flow rate ranges from 1:2 to 1:25.

8. The method of claim 1,
wherein at least some of the third stream is divided and recycled into the second stream.

9. A method for preparing acrylic acid, comprising:
obtaining lactic acid molecules by vaporizing the lactic acid according to the method of any one of claims 1 to 8;
subjecting lactic acid molecules to a dehydration reaction to prepare the acrylic acid; and
obtaining the acrylic acid.
